# EUROPÄISCHE PATENTANMELDUNG

(11) **EP 3 691 011 A1**
(43) Veröffentlichungstag der Anmeldung: **05.08.2020**
(21) Anmeldenummer: 19154112.7
(22) Anmeldetag: 29.01.2019
(51) Int. Cl.: H01M 8/18, C07D 237/32

(54) **DIHYDROXYPHTHALAZINE, IHRE HERSTELLUNG UND VERWENDUNG**

(71) Anmelder: Justus-Liebig-Universität Gießen, 35390 Gießen (DE)
(72) Erfinder: Wegner, Hermann A., 35435 Wettenberg (DE); Janek, Jürgen, 35392 Gießen (DE); Schröder, Daniel, 35390 Gießen (DE); Hong, Longcheng, Gießen, 35392 (CN); Hofmann, Jonas, 35606 Solms (DE); Schmalisch, Sebastian, 35396 Gießen (DE)
(74) Vertreter: Stumpf, Peter

(57) **Zusammenfassung**

Die vorliegende Erfindung umfasst Dihydroxyphthalazine, deren Herstellung, sowie Zubereitungen, die Dihydroxyphthalazine enthalten. Weiterhin betrifft die Erfindung die Verwendung von Dihydroxyphthalazinen bzw. diese enthaltende Zubereitungen als Katholyte in organischen Redox-Fluß-Batterien.

## Beschreibung

Die vorliegende Erfindung betrifft Dihydroxyphthalazine sowie ein Verfahren zu ihrer Herstellung und ihre Verwendung, insbesondere in wässrigen Redox-Flussbatterien, beispielsweise als Vorstufen für Diazanaphthochinone als Katholyt-Aktivmaterialien.

### Stand der Technik

Im Zuge einer nachhaltigen regenerativen Energieversorgung, insbesondere der nachhaltig-regenerativen Versorgung mit Elektrizität wird es zunehmend wichtig, den saisonal und mit hoher Fluktuation anfallenden Wind- und Solarstrom effektiv und sicher sowie langfristig zu speichern und bedarfsorientiert in das Stromnetz zu speisen. Dafür bieten sogenannte Redox-Flussbatterien ein enormes Potential, da sie vom prinzipiellen Aufbau her grundsätzlich sowohl hinsichtlich Leistungsstärke als auch Energiespeicherkapazität (synonym: Stromdichte und Strommenge) weitgehend beliebig skalierbar sind.

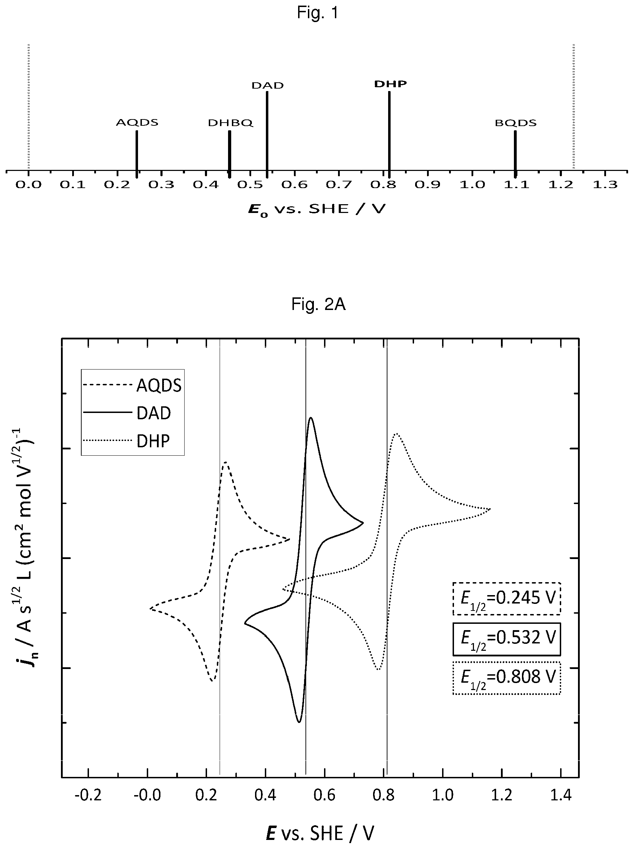

Die Entwicklung neuer Speichersysteme für elektrischen Strom ist somit eine der dringendsten Herausforderungen zur Umsetzung der angestrebten Energiewende. Insbesondere dem Elektrolyten, welcher sich aus Lösungsmittel, Aktivmaterial und gegebenenfalls Leitsalzen zur Erhöhung der elektrischen Leitfähigkeit zusammensetzt, kommt dabei eine entscheidende Rolle zu. Obwohl erste Prototypen mit metallbasierten wässrigen Elektrolyten bereits im Stand der Technik realisiert wurden, ist ein Ersatz dieser metallbasierten Systeme durch preiswertere und besser verfügbare organische Aktivmaterialien erforderlich. Die bisher im Stand der Technik bekannten organischen Moleküle, die als Aktivmaterialien eingesetzt werden, haben jedoch unter anderem die Nachteile einer sehr geringen Stabilität und eines eingeschränkt nutzbaren Potentialbereichs (vgl. Fig. 1).

Insbesondere organische Moleküle, die die notwendigen Voraussetzungen für die Verwendung aufseiten der positiven Batterieelektrode (Kathode) haben, sind bisher weitgehend unbekannt. 1,2-Benzochinon-3,5-disulfonsäure (BQDS) weist zwar mit einem exzellenten Redoxpotential, d.h. einer Elektronenaufnahme/-Abgabe bei besonders hohen Spannungen, an sich eine grundsätzlich gute Eignung als Katholyt-Aktivmaterial auf, ist jedoch sehr instabil und damit trotz des stark positiven Potentials nicht wirtschaftlich einsetzbar. Allgemein weisen die im Stand der Technik bekannten organischen Verbindungen für die positive Elektrodenseite entweder kein geeignetes Redoxpotential auf, was zu niedrigen Zellspannungen und in der Folge je nach Innenwiderstand zu ungenügenden Stromdichten führt, oder sind zu instabil für die langfristige technische Anwendung in Redox-Flussbatterien (bzw. kurz: "Flussbatterien"). Daher gibt es im Stand der Technik bis dato keine wirtschaftlich operierende Flussbatterie auf Basis organischer Verbindungen (Substanzen) im wässrigen Medium.

### Aufgabe

Aufgabe der vorliegenden Erfindung ist es Substanzen, bzw. chemische Verbindungen ("Verbindungen"/"Moleküle" - die Begriffe "Verbindung", "Molekül" sowie "Substanz" werden hier synonym verwendet -) sowie Zubereitungen solcher Substanzen/Verbindungen bereitzustellen, die ein für technische Anwendungen ausreichend hohes Redoxpotential aufweisen und darüber hinaus über eine ausreichende Langzeitstabilität verfügen, um direkt oder indirekt als Elektrolyte (Katholyte/Anolyte) in organischen Redox-Fluss-Batterien eingesetzt zu werden. Eine weitere Aufgabe der Erfindung besteht in der Bereitstellung von organischen Redox-Fluss-Batterien, die die genannten Substanzen/Verbindungen umfassen. Hier und im Folgenden werden die Begriffe "Redox-Fluss-Batterie" und "Flussbatterie" synonym verwendet. Weitere in der Literatur gebräuchliche Synonyme sind "Flow-Batterien" und "Redox-Flow-Batterien".

### Lösung der Aufgabe

Diese Aufgabe wird erfindungsgemäß gelöst durch die kennzeichnenden Merkmale des Hauptanspruchs sowie der nebengeordneten Ansprüche auf die Herstellung der Verbindungen, deren Verwendung für die Herstellung entsprechender Zubereitungen sowie die diese umfassenden Redox-Fluß-Batterien und deren Verwendung.

Im Rahmen der durch die Erfinder durchgeführten Studien zur Entwicklung von organischen Aktivmaterialien für wässrige Flussbatterien wurde überraschenderweise herausgefunden, dass im Kohlenstoff-Grundgerüst stickstoff-substituierte Hydrochinone, sogenannte Dihydroxyphthalazine, sowohl in einem günstigen Potentialbereich elektrochemisch aktiv sind, d.h. ein hohes Redoxpotential aufweisen, wodurch sie insbesondere für die Anwendung als positives Aktivmaterial im Katholyten von Redox-Flussbatterien geeignet sind, als auch über eine sehr gute chemische Stabilität für Langzeitanwendungen verfügen.

Als Aktivmaterial werden bekanntermaßen Substanzen verstanden, die redox-aktiv sind, d.h. in verschiedenen Oxidationsstufen vorliegen und elektrochemisch ineinander überführt werden können. Darunter fallen sowohl organische Redoxpaare (z. B. Chinon-Hydrochinon) als auch anorganische redoxaktive Metallspezies wie beispielsweise Fe²⁺/Fe³⁺. Je nach elektrochemischem Potential des Aktivmaterials kann dieses als Redoxmaterial im Katholyt (hohes Redoxpotential) oder im Anolyt (niedriges Redoxpotential) in der Vollzelle fungieren.

Als Katholyt wird im Sinne der hier offenbarten Erfindung der Elektrolyt, bzw. die Elektrolytlösung/Zubereitung, bezeichnet, der während des Entladevorgangs in direktem Einfluss der Kathode steht, d.h. den Kathodenraum der Flussbatterie durchströmt und das Aktivmaterial mit dem höheren Redoxpotential enthält. Als Anolyt wird im Sinne der hier offenbarten Erfindung analog der Elektrolyt, bzw. die Elektrolytlösung/Zubereitung, bezeichnet, der während des Entladevorgangs in direktem Einfluss der Anode steht, d.h. den Anodenraum der Flussbatterie durchströmt und das Aktivmaterial mit dem niedrigeren Redoxpotential enthält. Diese Festlegung ist nötig, da infolge der Stromumkehr zwischen dem Lade- und Entladevorgang definitionsgemäß auch Anode (Oxidation / Entnahme von Elektronen aus dem Elektrolyten) und Kathode (Reduktion / Abgabe von Elektronen an den Elektrolyten) ihre Plätze tauschen. Als übergeordneter Begriff für beide (Katholyt und Anolyt) wird der Begriff Elektrolyt, bzw. Elektrolytlösung, verwendet, wobei es sich aus dem jeweiligen Sachzusammenhang ergibt, für welchen der beiden Begriffe (Katholyt oder Anolyt) "Elektrolyt" bzw. "Elektrolytlösung" synonym verwendet wird.

Die bisher durchgeführten systematischen Untersuchungen an bzw. mit organischen Elektrolyten haben den überraschenden positiven Effekt der Stickstoff-Substitution im Kohlenstoff-Grundgerüst hinsichtlich Erhöhung des Redoxpotentials und Erhaltung der elektrochemischen Stabilität im Halbzellenmaßstab bestätigt.

Infolge der bisherigen Unkenntnis dieser positiven Eigenschaften von Dihydroxyphthalazinen im Stand der Technik wurden diese bisher naturgemäß auch nicht als Elektrolyte in Flussbatterien oder anderen elektrochemischen Anwendungen eingesetzt.

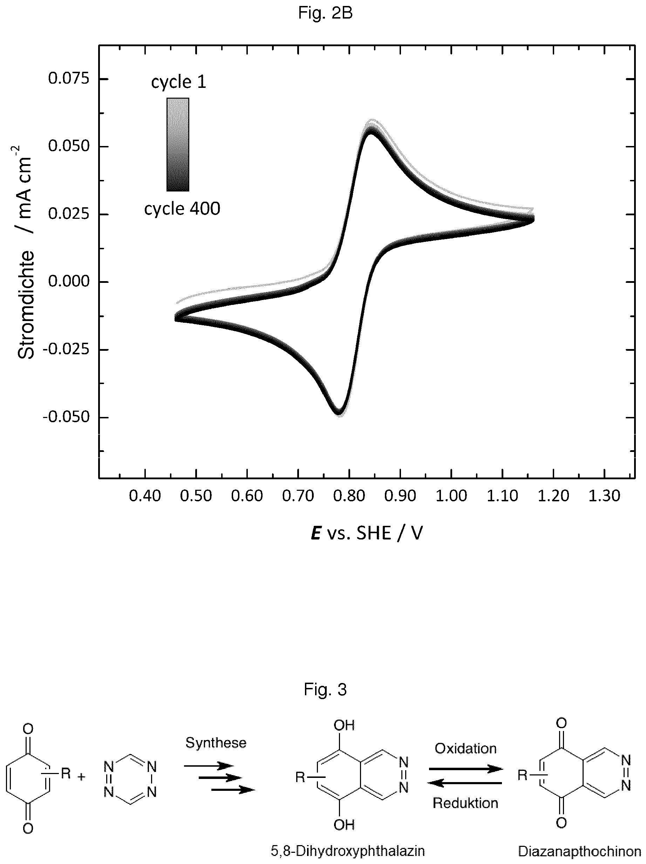

Fig. 3 zeigt beispielhaft den Zusammenhang zwischen den 5,8-Dihydroxyphthalazinen und den Diazanaphthochinonen, die in einem Redox-Gleichgewicht zueinander stehen. Es ist daraus ersichtlich, dass die Verwendung der Dihydroxyphthalazine gegenüber den Diazanaphthochinonen bei der Produktion von Flussbatterien bisher im Stand der Technik nicht beachtete massive Vorteile bietet, und demzufolge bisher auch im Stand der Technik für die Herstellung von Flussbatterien nicht verwendet werden, wie nachfolgend näher erläutert wird:

Dihydroxyphthalazine gehen im reduzierten Zustand aus der Synthese hervor, was dazu führt, dass das Molekül als vollaromatische Struktur eine besonders hohe Stabilität gegenüber potentiellen Nebenreaktionen aufweist. Zwar besteht durch den Kontakt mit Luftsauerstoff prinzipiell die Möglichkeit einer allmählichen Umwandlung der reduzierten chinoiden Verbindung in den oxidierten Ladungszustand, jedoch ist die Anwendung von Schutzgastechnologien seit langem üblich und mit geringem Aufwand und Kosten implementierbar. Lediglich das Anolyt-Aktivmaterial würde dementsprechend komplementär in seiner chinoiden und damit sauerstoff-stabileren Form eingesetzt. Da Katholyt-Aktivmaterialien aufgrund ihrer zweckgebundenen Elektronenaffinität im Vergleich zu deutlich elektronenreicheren Anolyt-Aktivmaterialien weitaus anfälliger für nukleophile Degradationsreaktionen sind, bietet die Synthese und Distribution der vollaromatischen Dihydroxyphthalazine enorme Vorteile im Hinblick auf Lagerbarkeit und Langzeitstabilität.

Aufgrund der besonderen Synthese von Dihydrophthalazinen auf Basis von Tetrazin und reichlich vorhandenen und nachhaltig erschließbaren chinoiden Präkursoren (vgl. Fig 3) - z. B. unter Verwendung von chinoiden Präkursoren aus Abfällen der Zellstoffindustrie, aus Lignozellulose o. dgl. - ist die Herstellung dieser Aktivmaterialien auch nach wirtschaftlichen Gesichtspunkten besonders attraktiv. So birgt der geringe Anschaffungspreis der Ausgangsmaterialien Potential für eine deutliche Senkung des Preises pro Kilowattstunde (insbesondere der Herstellungskosten) im Vergleich zu metallbasierten Flussbatterien. Das erfindungsgemäße Verfahren stellt somit neuartige Katholyte (Aktivmaterialien) durch ein kürzeres und damit kostengünstigeres und nachhaltigeres Verfahren bereit.

Die Erfindung befriedigt daher in überraschender und vielfältiger Weise ein seit langem im Stand der Technik bestehendes Bedürfnis zur Senkung der Herstellungs- und Betriebskosten von Flussbatterien: Durch kostengünstige und nachhaltige gezielte Herstellung und den Einsatz eines ab Synthese verfügbaren reduzierten, vollaromatischen Katholyten (z. B. Dihydroxyphthalazin) wird eine Flussbatterie erhalten, die selbst im Falle langer Standzeiten durch Ausschluss potentieller nukleophiler Nebenreaktionen des Katholyten ihre theoretische Kapazität aufrecht erhält. Die bereits seit langem bestehende technische Aufgabe der Vereinfachung bzw. Ökonomisierung der Herstellung von Flussbatterien wird somit überraschenderweise durch die hier offenbarte Erfindung gelöst.

Es ist für den Fachmann ohne weiteres erkennbar, dass der hier offenbarte Katholyt mit jedem beliebigen Anolyten kombiniert werden kann, ohne dadurch den Umfang der Erfindung oder ihrer Äquivalente zu verlassen. Beispielsweise kann die erfindungsgemäße Flussbatterie neben dem oben beschriebenen Katholyten als Anolyt umfassen: Organische Anolyt-Aktivmaterialien auf Basis von Anthrachinon, Naphthochinon, Diazaanthrachinon, Phenanthrenchinon, Alloxazin u. a. m., deren Redoxpotential sich ausreichend stark von dem Redoxpotential der erfindungsgemäßen Katholyte unterscheidet, oder anorganische Anolyte (Aktivmaterialien) auf Metallbasis, beispielsweise basierend auf V³⁺/V²⁺, Zn²⁺/Zn, Cr³⁺/Cr²⁺, Ti³⁺/Ti²⁺ u. a. m.

Ein weiterer Vorteil der Verwendung des Katholyten in seiner reduzierten Form (also beispielsweise als Dihydroxyphthalazin) besteht darin, dass die Hydroxylgruppen die Wasserlöslichkeit der reduzierten Form im Vergleich zu den Carbonylgruppen der oxidierten Form (z. B. Diazanaphthochinon) erhöht. Dies ändert zwar nichts daran, dass weiterhin die Löslichkeit der oxidierten Form der limitierende Faktor im Batteriebetrieb ist, es ist aber vorteilhafter bei der Herstellung der Flussbatterie bzw. der Lösung des Katholyten für die Flussbatterie, die reduzierte Form des Katholyten (Aktivmaterials) zu verwenden, da der Verfahrensschritt der Herstellung der Lösung mit der leichter löslichen reduzierten Form schneller und damit kostengünstiger erfolgt.

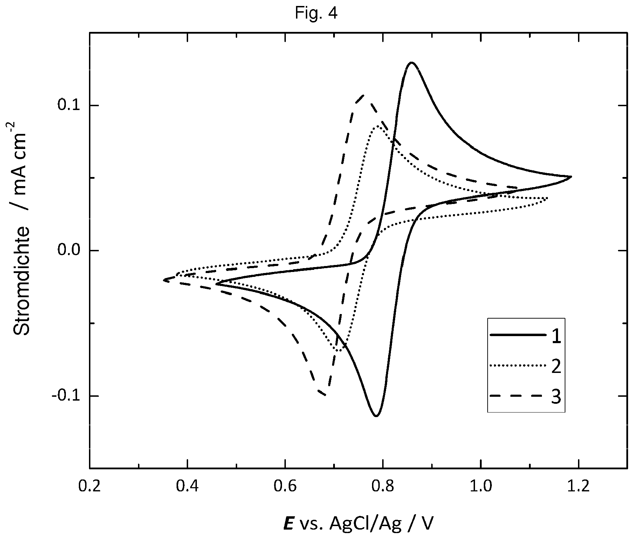

In Fig. 4 werden beispielhaft die vorteilhaften elektrochemischen Eigenschaften der 5,8-Dihydroxyphthalazine (DHPs) dargestellt. Insbesondere ist daraus beispielhaft der Einfluss der Substituenten am Benzolring ersichtlich: Elektronendonatoren wie beispielsweise Methoxygruppen und Methylgruppen induzieren eine negative Potentialverschiebung, d.h. das Potential, bei welchem das Aktivmaterial imstande ist, je nach Oxidationsstufe Elektronen aufzunehmen oder abzugeben, wird hin zu niedrigeren Werten verschoben, ausgedrückt in den Werten E_{1/2}, die jeweils bei dem Mittelwert des anodischen und kathodischen Stromflusses während einer Zyklovoltammetriemessung liegen. Für den Fachmann ist aus den offenbarten Beispielen ohne weiteres ersichtlich und naheliegend, dass die Einführung elektronenziehender Substituenten, d.h. Elektronenakzeptoren wie beispielsweise die Nitrogruppe - NO₂, am Benzolring in der Regel das elektrochemische Stabilitätsfenster analog zu höheren Werten verschieben. Es fällt daher in den Umfang der Erfindung sowie ihrer Äquivalente, den Benzolring sowohl mit elektronenschiebenden (d.h. Elektronendonatoren) als auch elektronenziehenden (d.h. Elektronenakzeptoren) Substituenten, oder beiden Varianten von Substituenten zu substituieren, um das elektrochemische Stabilitätsfenster in den für die jeweilige Anwendung gewünschten Bereich zu verschieben, d.h. zum Beispiel an das gewünschte Anodenpotential anzupassen. Dabei ist es unerheblich, ob der elektronenziehende oder elektronenschiebende Effekt auf einem +/- I-Effekt oder einem +/- M-Effekt beruht, wie das Beispiel von Methyl- und Methoxy-substituieren DHPs zeigt.

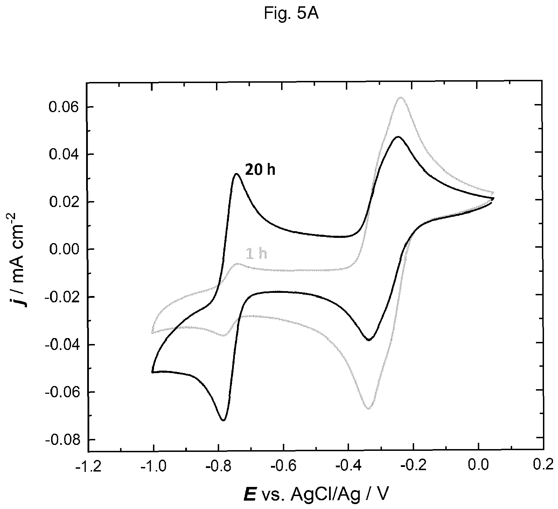

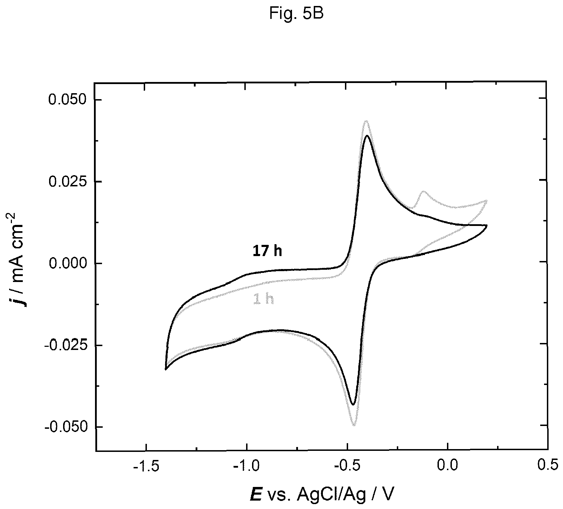

Anhand der in Fig. 5A und Fig. 5B dargestellten Messergebnisse von über einen längeren Zeitraum hinweg durchgeführten Zyklovoltammetriemessungen an jeweils einer Probe (Fig. 5A: Diazaanthrachinon, Fig. 5B: Dihydroxyphthalazin) wird beispielhaft die besonders vorteilhafte Stabilität der Dihydroxyphthalazin-basierten Redoxsysteme (z.B. 5,8-Dihydroxyphthalazine, DHPs) gegenüber Anthrachinon basierten Systemen (im Beispiel: Diazaanthrachinon) dargestellt.

Bei diesen Stabilitätsuntersuchungen dient die Änderung des über einen Zeitraum mehrerer Stunden dargestellten Verlaufs elektrochemischer Messergebnisse in Form von Zyklovoltammogrammen eines Aktivmaterials als Stabilitätskriterium. Dies ist in Fig. 5A beispielhaft anhand eines Diazaanthrachinons dargestellt, welches zur Illustration in definierten zeitlichen Abständen wiederholten Zyklovoltammetriemessungen unterzogen wurde. Je deutlicher sich die zeitlich versetzten Kurven voneinander unterscheiden, desto instabiler ist das untersuchte Redoxsystem, da sich die chemische Zusammensetzung innerhalb der Lösung im Laufe der Zeit verändert hat, d.h. redoxaktive Zersetzungsprodukte entstanden sind.

Diese vorstehenden Erläuterungen zur Auswertung bezüglich Fig. 5A gelten analog auch für das in Fig. 5B beispielhaft gezeigte Dihydroxyphthalazin-System, und aus dem Vergleich der beiden Abbildungen folgt somit unweigerlich die deutlich bessere Stabilität der Dihydroxyphthalzin-Systeme gegenüber den Diazaanthrachinon-Systemen in basischem Milieu (die Messungen in Fig. 5A/5B wurden in 1M KOH-Lösung vorgenommen): Bei dem erfindungsgemäßen Dihydroxyphthalzin-System haben sich nahezu keine elektrochemisch aktiven Zersetzungsprodukte gebildet, während solche bei dem Diazaanthrachinonsystem in größerem Umfange aufgetreten sind. Das auf 5,8-Dihydroxyphthalzin basierende Redoxsystem weist also eine deutlich größere elektrochemische Stabilität in basischem Milieu bis hin zu etwa pH = 14 oder mehr auf.

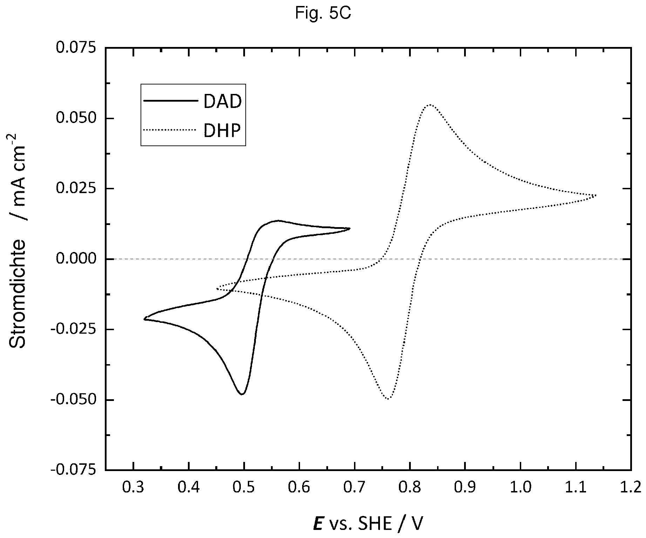

Analoge Messungen wurden auch in saurem Milieu vorgenommen, und auch hier zeigt sich anhand der in Fig. 5C beispielhaft dargestellten Messungen in saurer Lösung eine deutlich größere elektrochemische Stabilität der erfindungsgemäßen Dihydroxyphthalzin-Systeme gegenüber den Diazaanthrachinon-Systemen. Hier zeigt sich die Stabilität an der Symmetrie der Signale um die Nulllinie (vgl. dazu Legende zu Fig. 5C weiter unten), was - neben dem zuvor geschilderten Kriterium der Änderung des über einen Zeitraum von mehreren Stunden oder mehr dargestellten Verlaufs elektrochemischer Messergebnisse in Form von Zyklovoltammogrammen ein weiteres Stabilitätskriterium ist, das bevorzugt, aber nicht nur, in saurer Lösung verwendet wird. Insgesamt wurden Stabilitätsmessungen in folgenden Lösungen vorgenommen (berechnete pH-Werte in Klammern):
1M H₂SO₄ (pH = -0,26),
2M H₂SO₄ (pH = -0,84),
2M HCl (pH = (-0,69),
1M KOH (pH = 14).

In allen Fällen wurde eine deutlich erhöhte Stabilität der Dihydroxyphthalzin-Systeme gegenüber den Diazaanthrachinon-Systemen gefunden, woraus für den Fachmann ohne weiteres ersichtlich folgt, daß die überraschenderweise deutlich erhöhte Stabilität von Dihydroxyphthalzin-Systemen gegenüber Diazaanthrachinon-Systemen sowohl in diesem gesamten Bereich (von pH = -0,84 bis pH = 14 sowie auch jeweils über diese genannten Grenzwerte hinaus vorliegt.

Die hier bereits qualitativ deutlich erkennbare unterschiedliche Stabilität kann im Bedarfsfalle auch quantifiziert werden, beispielsweise indem die Fläche, die von den jeweiligen Signalen der aktiven Spezies umschlossen wird, als Maß für die elektrochemische Stabilität unter den gewählten Messbedingungen (z. B. Druck, Temperatur, Gasatmosphäre usw.) herangezogen wird (Fig. 5A / 5B). Analog kann (vgl. 5C) die Differenz der von den Signalen ober- und unterhalb der Nulllinie umschlossenen Flächen, ebenfalls als quantitatives Stabilitätskriterium dienen.

Dem Fachmann ist bekannt, dass die hier offenbarten Zahlenwerte (sowohl berechnete Zahlenwerte als auch Messergebnisse) den Umfang der Erfindung sowie ihrer Äquivalente nicht einschränken, sondern sich der Umfang der Erfindung und ihrer Äquivalente über die genannten Zahlenwerte hinaus erstreckt.

So umfassen beispielsweise Bereichsangaben stets alle - auch nicht explizit genannte - innerhalb der genannten Intervallgrenzen liegenden Zwischenwerte und alle denkbaren Teilintervalle. Genannte Bereichsgrenzen umfassen stets auch benachbarte Werte jenseits der angegebenen Bereichsgrenze; beispielsweise bei der Angabe der Raumtemperatur ("15 - 25 °C") ist dem Fachmann ohne weiteres ersichtlich, dass außerhalb in der Nähe des genannten Bereiches befindliche Werte mit umfasst sind. Das Gesagte gilt ebenfalls analog für Bereichsangaben von Formelindices, d.h. angegebene Indexbereiche umfassen immer auch sämtliche darin enthaltenen Teilintervalle sowie ggf. angrenzende Bereiche, auch wenn diese nicht explizit genannt sind. Die Angabe "C₁₋₆ Alkyl" umfasst z. B. - neben allen evtl. weiteren möglichen Teilbereichen - auch die hier beispielhaft aufgeführten Teilbereiche "C₁₋₂ Alkyl", "C₁₋₃ Alkyl", "C₁₋₄ Alkyl", "C₁₋₅ Alkyl", "C₂₋₃ Alkyl", "C₂₋₄ Alkyl", "C₂₋₅ Alkyl", "C₂₋₆ Alkyl", "C₃₋₄ Alkyl", "C₃₋₅ Alkyl", "C₃₋₆ Alkyl", "C₄₋₅ Alkyl" , "C₄₋₆ Alkyl" und "C₅₋₆ Alkyl" sowie ggf. den angrenzenden benachbarten Bereich "C₆₋₈ Alkyl".

Zusammenfassend läßt sich der Gegenstand der Erfindung wie folgt beschreiben:
Die Erfindung umfasst eine Verbindung, bzw. Verbindungen, der allgemeinen Formel I, 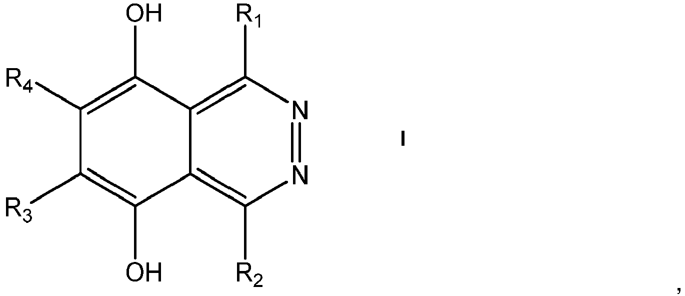 die dadurch gekennzeichnet ist, bzw. sind, dass die Substituenten R₁ bis R₄ unabhängig voneinander ausgewählt sind aus der Liste umfassend H, C₁₋₆ Alkyl, substituiertes C₁₋₆ Alkyl, F, Cl, Br, I, OH, C₁₋₆ Alkoxy, substituiertes C₁₋₆ Alkoxy, SO₃H, SO₃Rx, SO₂H, SO₂Rₓ, NH₂, NHRₓ, NRₓR_{y}, NO, NO₂, CO₂H, CO₂R_{z}, CO, Phosphoryl, Phosphonyl, Thiol, C₁₋₆ Alkylester, substituiertes C₁₋₆ Alkylester, C₁₋₆ Thioalkyl, substituiertes C₁₋₆ Thioalkyl, wobei die Reste Rₓ, R_{y} und R_{z} unabhängig voneinander ausgewählt sind aus der Liste umfassend C₁₋₆ Alkyl, substituiertes C₁₋₆ Alkyl, C₃₋₆ iso-Alkyl, substituiertes C₃₋₆ *iso*-Alkyl, C₄₋₆ *tert*-Alkyl, substituiertes C₄₋₆ *tert*-Alkyl.

Die Erfindung umfasst weiterhin Verbindungen der vorgenannten Art, die dadurch gekennzeichnet sind, dass sie in protonierter oder in deprotonierter Form vorliegen.

Die Erfindung umfasst weiterhin Zubereitungen, die mindestens eine Verbindung der vorgenannten Art umfassen, wobei die mindestens eine Verbindung gemäß vorgenannter Art in Substanz, gelöst, emulgiert oder dispergiert, als Nano- oder Mikrogel in einem Elektrolytlösungsmittel vorliegt.

Die Erfindung umfasst weiterhin Zubereitungen der vorgenannten Art, umfassend weiterhin im Elektrolytlösungsmittel gelöste Leitsalze und/oder weitere Additive.

Die Erfindung umfasst weiterhin die Verwendung der Verbindung, bzw. Verbindungen, gemäß vorgenannter Art oder der Zubereitung, bzw. Zubereitungen, gemäß vorgenannter Art als Katholyt in einer Redox-Fluss-Batterie.

Die Erfindung umfasst weiterhin Redox-Fluss-Batterien umfassend mindestens einen Katholyten gemäß vorgenannter Art.

Die Erfindung umfasst weiterhin Redox-Fluss-Batterien gemäß vorgenannter Art, umfassend weiterhin
- einen organischen Anolyten, ausgewählt aus der Liste umfassend Anthrachinon, substituiertes Anthrachinon, Naphthochinon, substituiertes Naphthochinon, Diazaanthrachinon, substituiertes Diazaanthrachinon, Diazanaphthochinon, substituiertes Diazanaphthochinon oder
- einen anorganischen Anolyten, ausgewählt aus der Liste umfassend die Redoxpaare V⁵⁺/V⁴⁺, V³⁺/V²⁺, Fe³⁺/^{Fe2+}, Ce⁴⁺/^{Ce3+}, Zn²⁺/Zn, Cr³⁺/Cr²⁺, Ti³⁺/^{Ti2+}.

Die Erfindung umfasst weiterhin die Verwendung der Redox-Fluss-Batterien gemäß vorgenannter Art zur Speicherung von elektrischer Energie für mobile und stationäre Anwendungen, insbesondere für Einsätze auf dem Gebiet der Elektromobilität, als Speicher in Land-, Luft- und Wasserfahrzeugen, als stationäre Speicher für die Notstromversorgung, den Spitzenlastausgleich, und für die Zwischenspeicherung elektrischer Energie aus erneuerbaren Energiequellen, insbesondere auf dem Sektor der Photovoltaik und der Windkraft, oder aus Gas-, Kohle-, Biomasse-, Gezeiten- und Meereskraftwerken.

Die Erfindung umfasst weiterhin ein Verfahren zur Herstellung der Verbindungen der vorgenannten Art, das dadurch gekennzeichnet ist, dass es die folgenden Schritte aufweist:
a) Bereitstellung der Edukte substituiertes 1,4-Benzochinon und substituiertes 1,2,4,5-Tetrazin sowie des Katalysatorkomplexes in einem Lösungsmittel unter inerter Atmosphäre in einem Reaktionsgefäß, wobei der Anteil an Katalysatorkomplex 0,01 bis 0,5 Äquivalente beträgt, der Anteil an 1,4-Benzochinon 1,0 Äquivalent beträgt und der Anteil an 1,2,4,5-Tetrazin 1,0 bis 10,0 Äquivalente beträgt;
b) Zugabe von 1 bis 20 mL wasserfreiem Benzotrifluorid pro mmol Ansatzgröße zu dem Ansatz aus Schritt a);
c) Mischen und Erwärmen des gemäß Schritt b) ergänzten Ansatzes aus Schritt a) auf 40 bis 70 °C;
d) Verschließen des Reaktionsgefäßes mit dem gemäß den Schritten a) bis c) behandelten Ansatz gasdicht und druckfest;
e) Erwärmen des befüllten und verschlossenen Reaktionsgefäßes auf 80 bis 200 °C, bevorzugt auf 90 bis 150 °C, besonders bevorzugt auf 100 bis 120 °C und ganz besonders bevorzugt auf 110 °C unter fortgesetztem Durchmischen des Inhalts des Reaktionsgefäßes;
f) Abkühlen auf 15 bis 25 °C und Entfernen des Lösungsmittels und des überschüssigen substituierten 1,2,4,5-Tetrazins aus dem Reaktionsgefäß, wobei ein Rückstand entsteht;
g) Versetzen des aus Schritt f) erhaltenen Rückstandes mit einem Lösungsmittel, ausgewählt aus der Liste umfassend Dichlormethan, Trichlormethan, Tetrachlormethan;
h) Reinigen des gemäß Schritt g) gelösten Rückstandes durch Chromatographie, wobei ein Eluat entsteht;
i) Entfernung des Lösungsmittels aus dem aus Schritt h) erhaltenen Eluats zur Gewinnung des Rohproduktes;
j) Entfernung weiterer Verunreinigungen durch Behandlung des Rohproduktes mit einem Lösungsmittel oder Lösungsmittelgemisch bei 40 bis 100 °C, bevorzugt bei 50 bis 80 °C, besonders bevorzugt bei 60 bis 70 °C und Abtrennung des die Verunreinigungen enthaltenden Lösungsmittels oder Lösungsmittelgemisches, wodurch das Produkt erhalten wird;
k) Trocknung des zurückgebliebenen Produktes bei Normaldruck oder Unterdruck.

In Schritt a) des vorstehend beschriebenen erfindungsgemäßen Verfahrens werden als Katalysatorkomplex monodentate Lewissäuren wie zum Beispiel BX₃, oder auch bidentate Lewissäuren, wie beispielsweise 9,10-dimethyl-9,10-diboranthrazen in freier Form oder auch als Komplex mit Phathalazinen oder anderen Diazinen eingesetzt; bei den Resten X in BX₃ handelt es sich dabei um elektronenziehende Reste, beispielsweise (jedoch nicht darauf beschränkt) um Halogenide (X = F, Cl, Br, I).

### Ausführungsbeispiele

### Generelle Informationen

Alle für die folgenden Ausführungsbeispiele verwendeten Reagenzien und Lösungsmittel werden von Sigma-Aldrich, TCl, Acros oder Alfa Aesar bezogen und mit Ausnahme der 1,4-Benzochinone ohne Aufreinigung verwendet. Dem Fachmann ist bekannt, dass dies keine einschränkende Bezugsquellenliste darstellt, sondern Reagenzien und Lösungsmittel (allgemein: Chemikalien) auch von anderen Herstellern/Lieferanten und in anderen Qualitäten bezogen und verwendet werden können, ohne den Umfang der Erfindung oder ihrer Äquivalente zu verlassen.

Neben dem Dezimalkomma wird im Rahmen dieser Anmeldeschrift auch der Dezimalpunkt verwendet, da beispielsweise Ausdrucke von Messgeräten oftmals nur Dezimalpunkte verwenden. Sowohl im Fließtext als auch in den Abbildungen können daher Zahlenwerte (Dezimalzahlen) entweder mit Dezimalkomma oder Dezimalpunkt angegeben werden. Dem Fachmann ist aus dem jeweiligen Zusammenhang ohne weiteres ersichtlich, welche Zahlendarstellung im jeweils vorliegenden konkreten Falle gerade verwendet wird.

Die Benzochinone werden vor der Verwendung aus Cyclohexan umkristallisiert oder säulenchromatographisch auf gereinigt. Trockene Apparaturen und luftempfindliche Reaktionen werden unter Stickstoff als Schutzgas und mit trockenen Lösungsmitteln von Sigma-Aldrich durchgeführt. Andere Schutzgase wie beispielsweise Argon können ebenfalls verwendet werden. Auf einem Bruker DPX-NMR (400 MHz) oder einem anderen geeigneten Gerät werden die ¹H- und ¹³C-NMR Messungen bei 25°C durchgeführt. Das Solvenssignal oder Tetramethylsilan werden als Referenz für die chemischen Verschiebungen verwendet, welche in ppm (parts per million) angegeben wird. Kopplungskonstanten werden in Hertz (Hz) notiert. Die NMR-Lösungsmittel werden beispielsweise von Cambridge Isotope Laboratories, Inc. (Andover, MA, USA) bezogen. Multipletts werden -in der dem Fachmann bekannter Weise - als Spannweite der Mitte definiert und mit s = Singulett und d = Dublett bezeichnet. Dünnschichtchromatographische Untersuchungen werden beispielsweise mit Polygram® SIL G/UV₂₅₄ Polyester-Fertigfolien mit 200 µm Kiesegel 60 Schicht durchgeführt und mit einer CAMAG UV Cabinet dual wavelength, 254/366 nm UV-Lampe detektiert. Die Synthesen des bidentaten Bor- Lewissäurekatalysators und der 1,2,4,5-Tetrazine erfolgen beispielsweise nach literaturbekannten und daher dem Fachmann geläufigen Vorschriften.

### Allgemeine Vorschrift zur Synthese von 5,8-Dihydroxyphthalazinen

Ein ausgeheiztes, mit Stickstoff geflutetes Schlenkrohr mit Rührfisch wird mit dem Katalysatorkomplex (0.1 Äq.), dem 1,4-Benzochinon (1 Äq.) und 1,2,4,5-Tetrazin (5 Äq.) beladen und mit trockenen Benzotrifluorid (10 mL/mmol) versetzt. Das Reaktionsgemisch wird auf 55°C erwärmt, versiegelt (d.h. auch von der Schutzgas-Zufuhr abgetrennt) und für weitere 20 h auf 110°C erhitzt. Dem Fachmann ist bekannt, dass diese Versiegelung auf verschiedenste Arten erfolgen kann, ohne dadurch den Umfang der Erfindung oder ihrer Äquivalente zu verlassen, beispielsweise durch Schließen eines Hahnes oder Ventils, oder durch Abschmelzen des Reaktionsgefäßes. Es ist bevorzugt, jedoch nicht zwingend erforderlich, dass das Reaktionsgefäß inklusive Siegelverschluß mindestens dem Druckanstieg im versiegelten Reaktionsgefäß durch das Erhitzen auf 110°C widersteht.

Nach dem Abkühlen auf Raumtemperatur (Umgebungstemperatur), also beispielsweise auf 15 - 25 °C, wird das Lösungsmittel und der Überschuss Tetrazin mittels Vakuum und Kühlfalle entfernt. Der Rückstand wird mittels Dichlormethan/Methanol gelöst/suspendiert und auf Kieselgel aufgetragen. Die weitere Aufreinigung erfolgt via Säulenchromatografie (Kieselgel, Dichlormethan/Methanol 8:2 - 6:4) oder einem anderen geeigneten Trennungsverfahren. Nach dem Entfernen des Lösungsmittels, beispielsweise unter vermindertem Druck, werden die festen Rückstände zur weiteren Aufreinigung mit einem heißen Chloroform/Methanol-Gemisch (9:1) versetzt, anschließend wieder abgekühlt und abfiltriert. Der erhaltene Filterkuchen wird im Hochvakuum getrocknet und somit das 5,8-Dihydroxyphthalazin als Feststoff isoliert. Dem Fachmann ist bekannt, welcher Temperaturbereich für die Erhitzung der Chloroform/Methanol-Mischung (9:1) vorteilhafterweise gewählt werden sollte. Beispielsweise kann die Mischung (inklusive Filterkuchen) unter Rückfluß zum Sieden erhitzt und bis zu mehrere Stunden lang unter Rückfluß gehalten werden, ohne dabei den Umfang der Erfindung oder ihrer Äquivalente zu verlassen.

Folgende Dihydroxyphthalazine wurden mittels vorstehender allgemeiner Arbeitsvorschrift hergestellt und charakterisiert:

### 5,8-Dihydroxy-2,3-phthalazin:

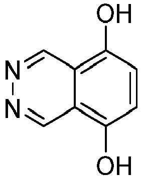

Isoliert als grüner Feststoff (Ausbeute: 25%).

¹H-NMR (400 MHz, DMSO-*d*₆) δ 10.27 (s, 2H), 9.57 (s, 2H), 7.16 (s, 2H).

¹³C-NMR (101 MHz, DMSO-*d*₆) δ 145.4, 144.9, 116.4, 116.3.

HRMS (ESI-MS) m/z berechnet [C₈H₆N₂O₂]⁺ / [H]⁺ (Molionenpeak + 1): 163.0502; gefunden: 163.0502.

### 5,8-Dihydroxy-6,7-dimethyl-2,3-phthalazin ("DHP(Me)₂"):

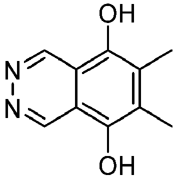

Isoliert als brauner Feststoff (Ausbeute: 14%).

¹H-NMR (400 MHz, DMSO-*d*₆) δ 9.62 (s, 2H), 9.33 (s, 2H), 2.33 (s, 6H).

¹³C-NMR (101 MHz, DMSO-*d*₆) δ 146.0, 142.9, 129.5, 116.1, 13.9.

HRMS (ESI-MS) m/z berechnet [C₁₀H₁₀N₂O₂]⁺ / [H]⁺ (Molionenpeak + 1): 191.0815; gefunden: 191.0818.

### 6-Chloro-5,8-dihydroxy-2,3-phthalazin:

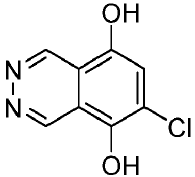

Isoliert als dunkelroter Feststoff (Ausbeute: 31%)

¹H-NMR (400 MHz, DMSO-*d*₆) δ 10.89 (s, 1H), 10.28 (s, 1H), 9.71 (d, *J* = 1.5 Hz, 1H), 9.59 (d, *J* = 1.5 Hz, 1H), 7.22 (s, 1H).

¹³C NMR (101 MHz, DMSO-*d*₆) δ 146.8, 145.6, 144.3, 140.6, 122.4, 118.4, 116.6, 116.0.

HRMS (ESI-MS) m/z berechnet [C₈H₅ClN₂O₂]⁺ / [H]⁺ (Molionenpeak + 1): 197,0113; gefunden: 197,0117.

### 5,8-Dihydroxy-6-methoxy-2,3-phthalazin ("DHP(MeO)"):

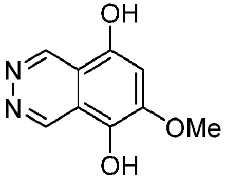

Isoliert als dunkelroter Feststoff (Ausbeute: 32%)
¹H-NMR (400 MHz, DMSO-*d*₆) δ 10.61 (s, 1H), 9.53 (d, *J* = 1.4 Hz, 1H), 9.41 (d, *J* = 1.4 Hz, 14H), 9.36 (s, 1H), 7.14 (s, 1H), 3.92 (s, 3H).

¹³C-NMR (101 MHz, DMSO-*d*₆) δ 149.0, 147.3, 145.5, 145.03,132.01, 116.8, 110.5, 103.9, 56.3.

HRMS (ESI-MS) m/z calculated for [C₉H₈N₂O₂]⁺ / [H]⁺ (Molionenpeak + 1): 163,0502 ; gefunden: 163,0503.

### Elektrochemische Untersuchungen

Die hervorragenden elektrochemischen Eigenschaften von Dihydroxyphthalazinen werden exemplarisch anhand einer Gegenüberstellung der Ladungstransfercharakteristiken strukturverwandter Verbindungen verdeutlicht (vgl. Fig. 2A). Hierzu wird das Redox-Verhalten verschiedener Elektrolyte mittels Zyklovoltammetrie in einer Dreielektrodenanordnung untersucht. Für den Elektrolyten wird - sofern nicht anders angegeben - 2M HCl als Lösungsmittel verwendet, in welchem das zu untersuchende Aktivmaterial in 0.5 mM Konzentration gelöst ist. Als Arbeitselektrode wird Glaskohlenstoff verwendet, wohingegen die Gegenelektrode aus Platin besteht. Die Potentialmessung erfolgt gegen eine Ag/AgCl-Referenz, deren Werte mittels Umrechnungsfaktor (+0.21 V) auf die Potentialskala der Standardwasserstoffelektrode (SHE) übertragen werden. Wie in Fig. 3 zu erkennen, führt bereits die Stickstofffunktionalisierung der Anthrachinonbasis (AQDS) zu einer deutlichen positiven Verschiebung des Redoxpotentials (DAD) um etwa 285 mV. Durch eine gezielte Abwandlung der Basisstruktur (DNQ) kann die Elektronenaffinität und somit das Redoxpotential der Substanz erneut deutlich erhöht werden, sodass eine Verbindung entsteht, welche aufgrund ihrer Aktivität bei einem hohen Potential von 0.8 V (vs. SHE) als Katholyt-Aktivmaterial eingesetzt werden kann. Zum Nachweis der Langzeitstabilität der erfindungsgemäßen Verbindungen und der Reversibilität des stattfindenden Ladungstransfers wird der Elektrolyt beispielhaft unter den vorstehend genannten Bedingungen einer 400 Zyklen umfassenden Zyklovoltammetriemessung mit einer Gesamtdauer von 15 Stunden unterzogen (vgl. Fig. 2B). Innerhalb dieses Zeitraums wird anhand der Messwerte keine relevante Veränderung des Redox-Verhaltens beobachtet, was die herausragende Zyklenstabilität der Verbindungsklasse der Dihydroxyphthalazine im Halbzellenmaßstab belegt.

Zur weiteren Anpassung der elektrochemischen Eigenschaften können weiterführende Funktionalisierungen an der Dihydroxyphthalazin-Basisstruktur durchgeführt werden (siehe z. B. weiter oben). Durch gezielte Substitution mit Gruppen verschiedener Elektronenaffinität kann das Redoxpotential in positiver wie in negativer Richtung anwendungsbezogen eingestellt werden (vgl. Fig. 4). Im Falle der dargestellten Substitutionsmuster wird das Redoxpotential beispielhaft ausgehend von der unsubstituierten Verbindung durch elektronenreiche Substituenten um mehr als 100 mV in negativer Richtung verschoben. Im Falle dieser Messungen (Fig. 4) beträgt die Aktivmaterialkonzentration 1 mM.

### Abbildungslegenden

- Fig. 1:: Beispielhafte Übersicht der Redoxpotentiale verschiedener chinoider Aktivmaterialien in 1M H₂SO₄. Die gestrichelten Linien markieren den elektrochemischen Stabilitätsbereich von Wasser, welches in wässrigen Flussbatterien als Lösungsmittel dient und den zugänglichen Potentialbereich vorgibt. Die dargestellten Potentiale von AQDS, DHBQ, BQDS und DAD wurden der dem Fachmann bekannten Fachliteratur entnommen; der Wert für DHP wurde messtechnisch experimentell ermittelt.
- Fig. 2A:: Beispielhaft ausgewähltes Zyklovoltammogramm des unsubstituierten Dihydroxyphthalazins sowie der strukturähnlichen Verbindungen AQDS und DAD zur Darstellung des jeweiligen Potentialbereichs elektrochemischer Aktivität. DAD und DNQ sind bei dieser beispielhaften Zusammenstellung in 2M HCl gemessen, AQDS in 2M H₂SO₄.
- Fig. 2B:: Stabilitätstest des unsubstituierten Dihydroxyphthalazins (Ausführungsbeispiel). Mithilfe von Zyklovoltammetrie wurden insgesamt 400 Messzyklen durchlaufen, die sich infolge ihrer Ähnlichkeit fast gänzlich überlappen. Aus der Deckungsgleichheit der einzelnen Messzyklen ist ersichtlich, dass der stattfindende Ladungstransferprozess eine hohe Reversibilität aufweist und über den betrachteten Zeitraum von 15 Stunden (400 Messzyklen) nahezu unverändert abläuft, was eine hohe Langzeitstabilität beweist.
- Fig. 3:: Beispielhafte Darstellung des Zusammenhangs zwischen Syntheseweg zu den 5,8-Dihydroxyphthalazinen (bzw. 5,8-Phthalazindiolen) und deren Redox-Gleichgewicht zu den Diazanaphthochinonen (bzw. 6,7-Diaza1,4-naphthalendionenen oder auch 5,8-Phthalazindionen).
- Fig. 4:: Zyklovoltammogramme verschiedener DHP-Derivate (Ausführungsbeispiele) zur Verdeutlichung der Variabilität des Redoxpotentials. Legende: Kurve 1 = DHP (E_{1/2} = 0,612 V); Kurve 2 = DHP(MeO) (E_{1/2} = 0,539 V); Kurve 3 = DHP(Me)₂ (E_{1/2} = 0,508 V).
- Fig. 5A:: Beispielhafte graphische Darstellung der elektrochemischen Stabilität von Diazaanthrachinon-basierten Redoxsystemen, untersucht durch wiederholte Cyclovoltammetriemessungen in 1M KOH über einen Zeitraum von 20 Stunden. Die exemplarischen Kurven, welche nach 1 h und 20 h Verweilzeit des Aktivmaterials in Lösung aufgezeichnet wurden, sind durch eine entsprechende Beschriftung gekennzeichnet. Dem Fachmann ist ersichtlich, dass das für Diazaanthrachinon charakteristische Signal bei -0.3 V über den beobachteten Zeitraum von 20 h deutlich an Intensität verliert. Das Anwachsen des Signals bei -0.75 V hingegen veranschaulicht die Zunahme des aus der Degradation des Ausgangsstoffs resultierenden, elektrochemisch aktiven Zersetzungsprodukts. Die betreffenden Signale nach 1 h Verweilzeit und nach 20 h Verweilzeit weisen jeweils eine große Differenz zueinander auf.
- Fig. 5B:: Beispielhafte graphische Darstellung der elektrochemischen Stabilität von 5,8-Dihydroxyphthalzin-basierten Redoxsystemen, untersucht durch wiederholte Cyclovoltammetriemessungen in 1M KOH über einen Zeitraum von 17 Stunden. Die exemplarischen Kurven, welche nach 1 h und 17 h Verweilzeit des Aktivmaterials in Lösung aufgezeichnet wurden, sind durch eine entsprechende Beschriftung gekennzeichnet. Das dem 5,8-Dihydroxyphthalzin zuzuordnende Signal bei -0.4 V erfährt über die Messdauer von 17 h lediglich eine marginale Reduzierung im Vergleich zur Messung zu Beginn der Exposition. Die geringe Differenz beider Signale nach jeweils 1 h Verweilzeit und 17 h Verweilzeit ist als Maß für die hohe elektrochemische Stabilität der Verbindung zu werten.
- Fig. 5C:: Beispielhafte graphische Darstellung der größeren elektrochemischen Stabilität von 5,8-Dihydroxyphthalzin-basierten Redoxsystemen im Vergleich zu DAD (Diazaanthrachinon), untersucht durch Cyclovoltammetriemessungen von DHP und DAD in saurer Lösung (im vorliegenden Beispiel: 1M Schwefelsäure): Das gewählte Beispiel DHP ist zum Beispiel in Schwefelsäure-Lösung deutlich stabiler, was sich an der Symmetrie der Signale um die horizontale Nulllinie (gestrichelt) zeigt. Im Falle des Diazaanthrachinons (DAD, durchgezogene Linie) kann nur ein Bruchteil der während der Reduktion (unterhalb der Nulllinie) übertragenen Ladung wieder entnommen werden (Oxidation; oberhalb der Nulllinie). Im Falle von DHP (punktierte Linie) liegt dagegen ein reversibler Ladungstransfer vor (Verhältnis Oxidation:Reduktion = 1:1).

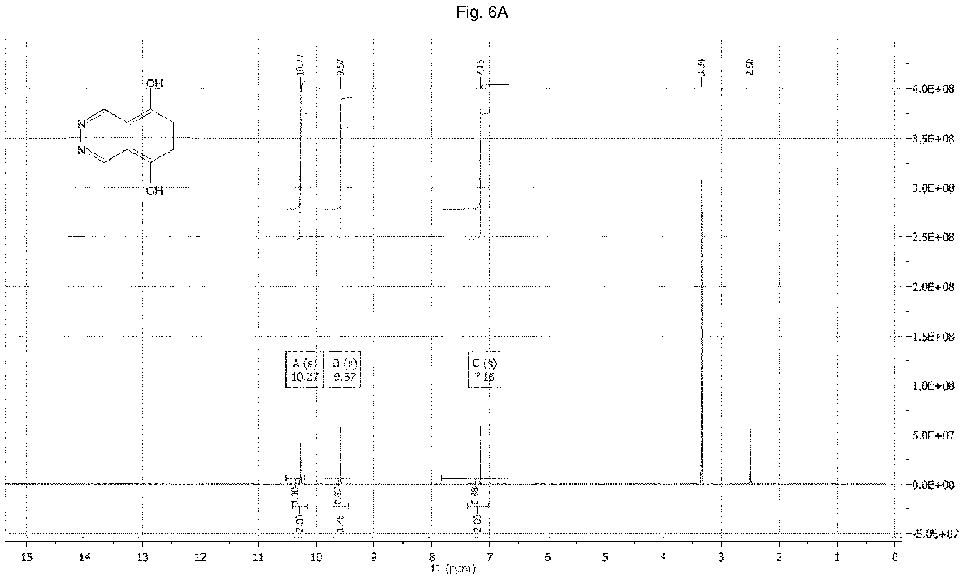

- Fig. 6A:: ¹H-NMR-Spektrum von 5,8-Dihydroxy-2,3-phthalazin. Dem Fachmann ist die Bedeutung der Abszissen- und Ordinatenbeschriftung sowie der Zahlen- und sonstigen Angaben (z.B. Formelbild, Spinsystem, Integrale und Positionsangaben zu den Signalen) in dieser und allen weiteren Zeichnungen bekannt und bedarf daher keiner näheren Erläuterung.

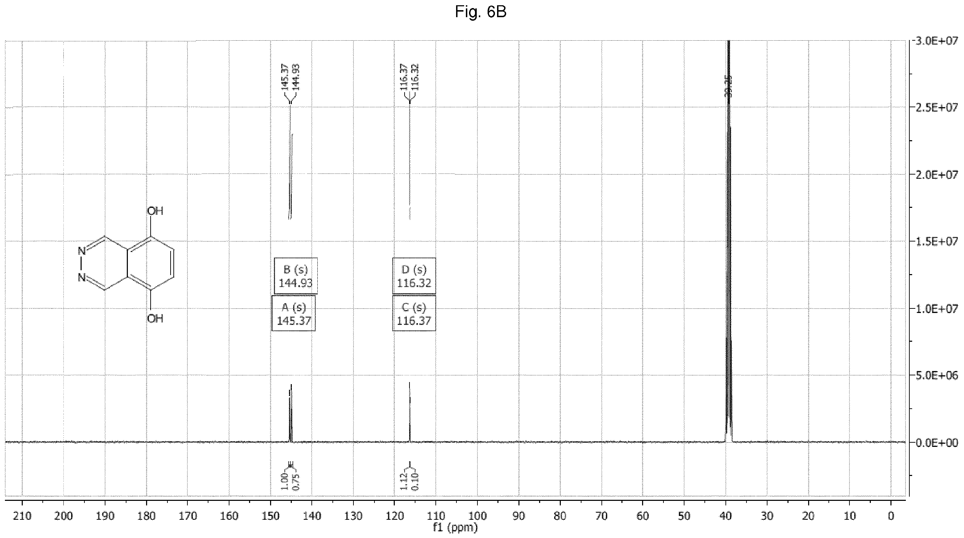

- Fig. 6B:: ¹³C-NMR-Spektrum von 5,8-Dihydroxy-2,3-phthalazin.

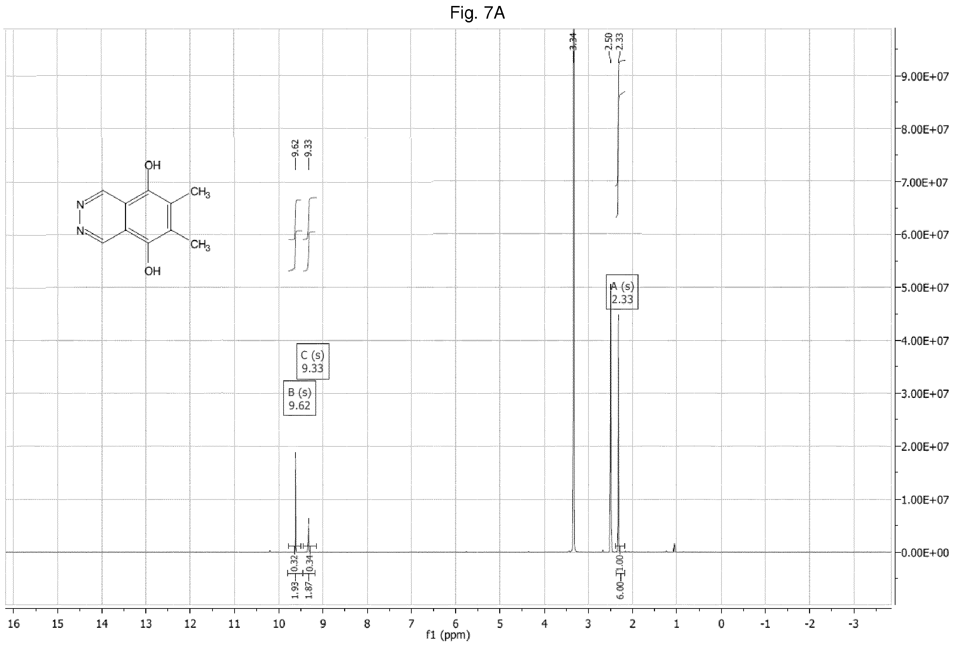

- Fig. 7A:: ¹H-NMR-Spektrum von 5,8-Dihydroxy-6,7-dimethyl-2,3-phthalazin.

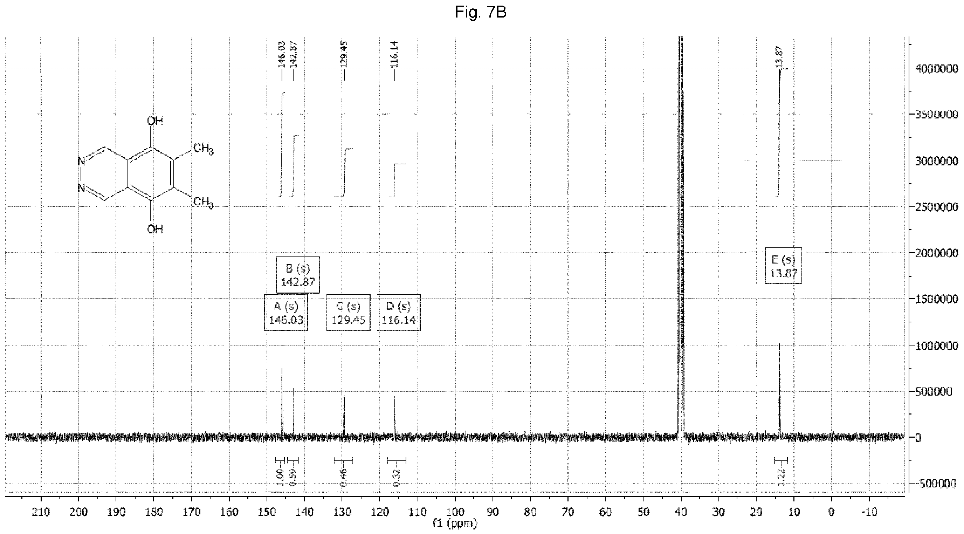

- Fig. 7B:: ¹³C-NMR-Spektrum von 5,8-Dihydroxy-6,7-dimethyl-2,3-phthalazin.

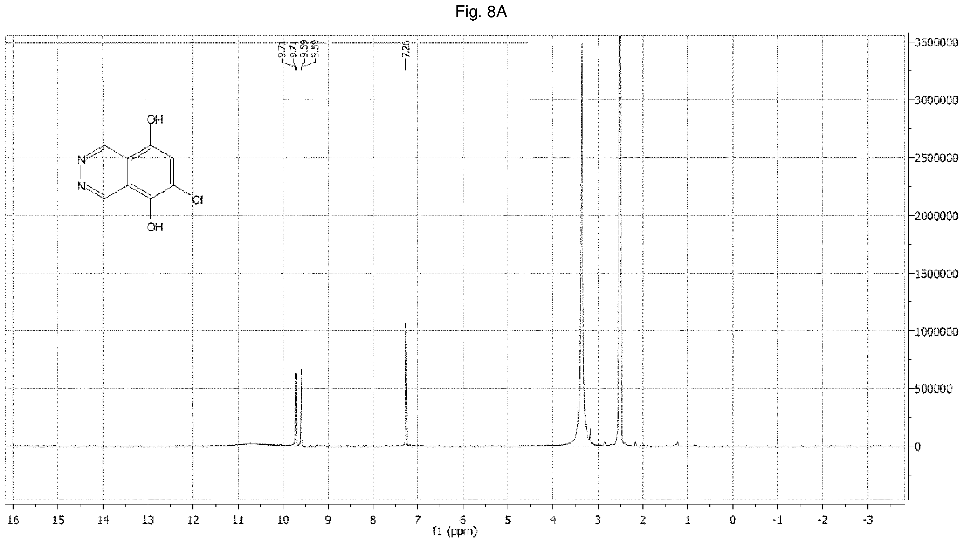

- Fig. 8A:: ¹H-NMR-Spektrum von 6-Chloro-5,8-dihydroxy-2,3-phthalazin.

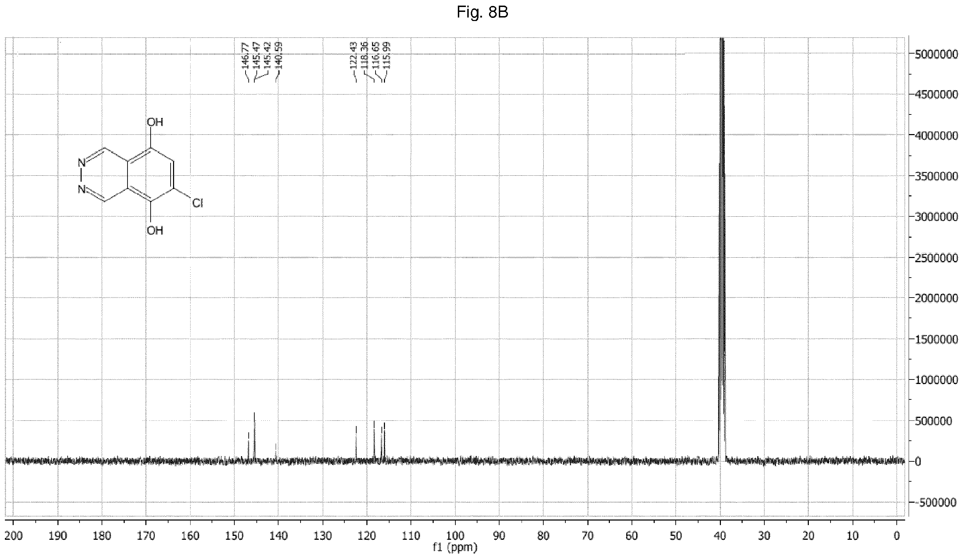

- Fig. 8B:: ¹³C-NMR-Spektrum von 6-Chloro-5,8-dihydroxy-2,3-phthalazin.

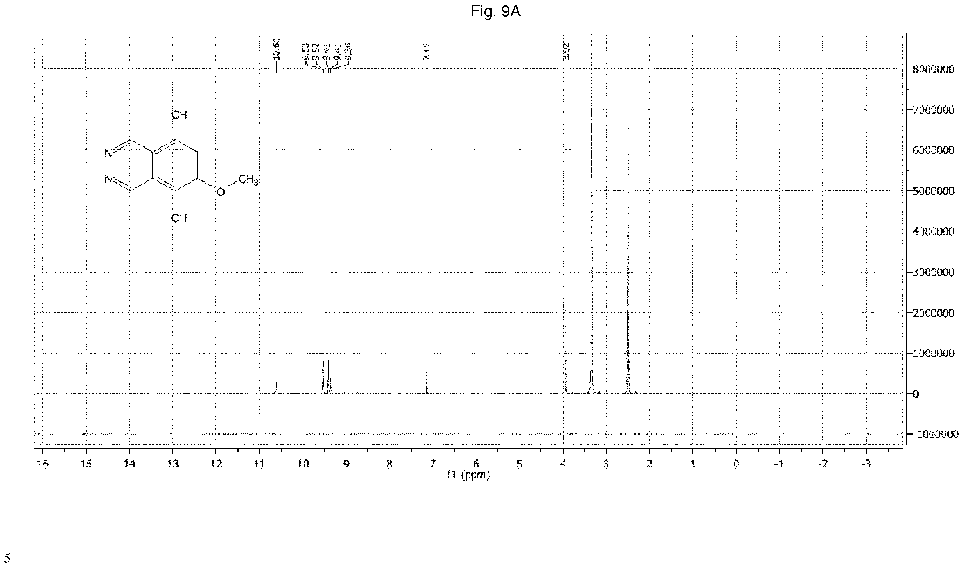

- Fig. 9A:: ¹H-NMR-Spektrum von 5,8-Dihydroxy-6-methoxy-2,3-phthalazin.

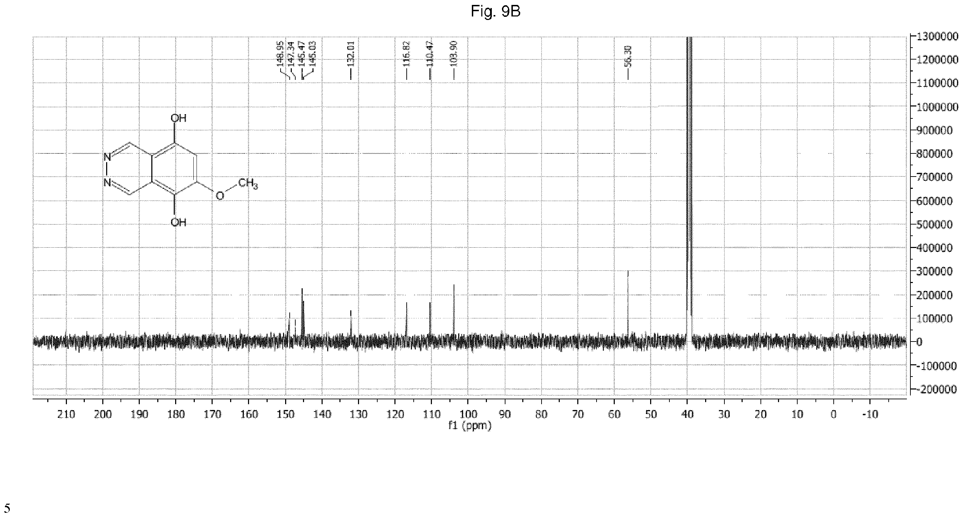

- Fig. 9B:: ¹³C-NMR-Spektrum von 5,8-Dihydroxy-6-methoxy-2,3-phthalazin.

### Bezugszeichen(Abkürzungs-)liste

Neben den im allgemeinen Sprachgebrauch sowie im wissenschaftlichen Sprachgebrauch üblichen Abkürzungen und Akronymen werden im Rahmen dieser Anmeldung u.a. noch folgende Abkürzungen/Akronyme verwendet, wobei diese Liste nicht abschließend zu verstehen ist.
- BQDS: 1,2-Benzochinon-3,5-disulfonsäure
- DAD: Diazaanthrachinon
- AQDS: Anthrachinondisulfonsäure
- DNQ: Diazanaphthachinon
- DHP: Dihydroxyphthalazin
- SHE: Standard-Wasserstoffelektrode

## Patentansprüche

1. Verbindung der allgemeinen Formel I, 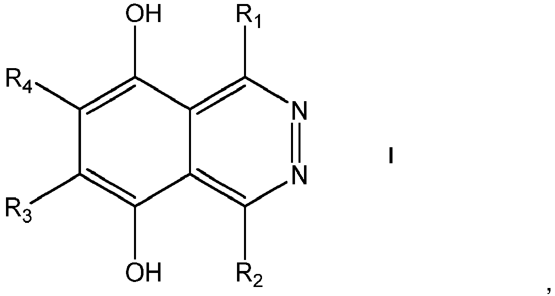 **dadurch gekennzeichnet, dass** die Substituenten R₁ bis R₄ unabhängig voneinander ausgewählt sind aus der Liste umfassend H, C₁₋₆ Alkyl, substituiertes C₁₋₆ Alkyl, F, Cl, Br, I, OH, C₁₋₆ Alkoxy, substituiertes C₁₋₆ Alkoxy, SO₃H, SO₃Rₓ, SO₂H, SO₂Rₓ, NH₂, NHRₓ, NRₓR_{y}, NO, NO₂, CO₂H, CO₂R_{z}, CO, Phosphoryl, Phosphonyl, Thiol, C₁₋₆ Alkylester, substituiertes C₁₋₆ Alkylester, C₁₋₆ Thioalkyl, substituiertes C₁₋₆ Thioalkyl, wobei die Reste Rₓ, R_{y} und R_{z} unabhängig voneinander ausgewählt sind aus der Liste umfassend C₁₋₆ Alkyl, substituiertes C₁₋₆ Alkyl, C₃₋₆ iso-Alkyl, substituiertes C₃₋₆ *iso-*Alkyl, C₄₋₆ *tert*-Alkyl, substituiertes C₄₋₆ *tert*-Alkyl.

2. Verbindung gemäß Anspruch 1, **dadurch gekennzeichnet, dass** sie in protonierter oder in deprotonierter Form vorliegt.

3. Zubereitung umfassend mindestens eine Verbindung gemäß einem der Ansprüche 1 bis 2, wobei die mindestens eine Verbindung gemäß Ansprüchen 1 bis 2 in Substanz, gelöst, emulgiert oder dispergiert, als Nano- oder Mikrogel in einem Elektrolytlösungsmittel vorliegt.

4. Zubereitung gemäß Anspruch 3 umfassend weiterhin im Elektrolytlösungsmittel gelöste Leitsalze und/oder weitere Additive.

5. Verwendung der Verbindung gemäß einem der Ansprüche 1 bis 2 oder der Zubereitung gemäß einem der Ansprüche 3 bis 4 als Katholyt in einer Redox-Fluss-Batterie.

6. Redox-Fluss-Batterie umfassend mindestens einen Katholyten gemäß Anspruch 5.

7. Redox-Fluss-Batterie gemäß Anspruch 6 umfassend weiterhin
- einen organischen Anolyten, ausgewählt aus der Liste umfassend Anthrachinon, substituiertes Anthrachinon, Naphthochinon, substituiertes Naphthochinon, Diazaanthrachinon, substituiertes Diazaanthrachinon, Diazanaphthochinon, substituiertes Diazanaphthochinon oder
- einen anorganischen Anolyten, ausgewählt aus der Liste umfassend die Redoxpaare V⁵⁺/^{V4+}, V³⁺/V²⁺, Fe³⁺/^{Fe2+}, Ce⁴⁺/^{Ce3+}, Zn²⁺/Zn, Cr³⁺/Cr²⁺, Ti³⁺/^{Ti2+}.

8. Verwendung der Redox-Fluss-Batterie gemäß Anspruch 6 zur Speicherung von elektrischer Energie für mobile und stationäre Anwendungen, insbesondere für Einsätze auf dem Gebiet der Elektromobilität, als Speicher in Land-, Luft- und Wasserfahrzeugen, als stationäre Speicher für die Notstromversorgung, den Spitzenlastausgleich, und für die Zwischenspeicherung elektrischer Energie aus erneuerbaren Energiequellen, insbesondere auf dem Sektor der Photovoltaik und der Windkraft, oder aus Gas-, Kohle-, Biomasse-, Gezeiten- und Meereskraftwerken.

9. Verfahren zur Herstellung der Verbindung gemäß Anspruch 1, **dadurch gekennzeichnet, dass** es die folgenden Schritte aufweist:
a) Bereitstellung der Edukte substituiertes 1,4-Benzochinon und substituiertes 1,2,4,5-Tetrazin sowie des Katalysatorkomplexes in einem Lösungsmittel unter inerter Atmosphäre in einem Reaktionsgefäß, wobei der Anteil an Katalysatorkomplex 0,01 bis 0,5 Äquivalente beträgt, der Anteil an 1,4-Benzochinon 1,0 Äquivalent beträgt und der Anteil an 1,2,4,5-Tetrazin 1,0 bis 10,0 Äquivalente beträgt;
b) Zugabe von 1 bis 20 mL wasserfreiem Benzotrifluorid pro mmol Ansatzgröße zu dem Ansatz aus Schritt a);
c) Mischen und Erwärmen des gemäß Schritt b) ergänzten Ansatzes aus Schritt a) auf 40 bis 70 °C;
d) Verschließen des Reaktionsgefäßes mit dem gemäß den Schritten a) bis c) behandelten Ansatz gasdicht und druckfest;
e) Erwärmen des befüllten und verschlossenen Reaktionsgefäßes auf 80 bis 200 °C, bevorzugt auf 90 bis 150 °C, besonders bevorzugt auf 100 bis 120 °C und ganz besonders bevorzugt auf 110 °C unter fortgesetztem Durchmischen des Inhalts des Reaktionsgefäßes;
f) Abkühlen auf 15 bis 25 °C und Entfernen des Lösungsmittels und des überschüssigen substituierten 1,2,4,5-Tetrazins aus dem Reaktionsgefäß, wobei ein Rückstand entsteht;
g) Versetzen des aus Schritt f) erhaltenen Rückstandes mit einem Lösungsmittel, ausgewählt aus der Liste umfassend Dichlormethan, Trichlormethan, Tetrachlormethan;
h) Reinigen des gemäß Schritt g) gelösten Rückstandes durch Chromatographie, wobei ein Eluat entsteht;
i) Entfernung des Lösungsmittels aus dem aus Schritt h) erhaltenen Eluats zur Gewinnung des Rohproduktes;
j) Entfernung weiterer Verunreinigungen durch Behandlung des Rohproduktes mit einem Lösungsmittel oder Lösungsmittelgemisch bei 40 bis 100 °C, bevorzugt bei 50 bis 80 °C, besonders bevorzugt bei 60 bis 70 °C und Abtrennung des die Verunreinigungen enthaltenden Lösungsmittels oder Lösungsmittelgemisches, wodurch das Produkt erhalten wird;
k) Trocknung des zurückgebliebenen Produktes bei Normaldruck oder Unterdruck.
